(19) Europäisches Patentamt
European Patent Office
Office européen des brevets

(11) Veröffentlichungsnummer : **0 304 613 B1**

# (12) EUROPÄISCHE PATENTSCHRIFT

(45) Veröffentlichungstag der Patentschrift : **26.06.91 Patentblatt 91/26**

(51) Int. Cl.⁵ : **C07D 311/58,** B41M 5/124, B41M 5/26, C07D 405/04

(21) Anmeldenummer : **88111573.7**

(22) Anmeldetag : **19.07.88**

(54) **Benzopyranderivate.**

(30) Priorität : **25.07.87 DE 3724757**

(43) Veröffentlichungstag der Anmeldung : **01.03.89 Patentblatt 89/09**

(45) Bekanntmachung des Hinweises auf die Patenterteilung : **26.06.91 Patentblatt 91/26**

(84) Benannte Vertragsstaaten : **CH DE FR GB IT LI**

(56) Entgegenhaltungen :
**EP-A- 0 236 973**
**FR-A- 2 156 909**

(73) Patentinhaber : **BASF Aktiengesellschaft
Carl-Bosch-Strasse 38
W-6700 Ludwigshafen (DE)**

(72) Erfinder : **Guentner, Andreas, Dr.
Carl-Bosch-Ring 20
W-6710 Frankental (DE)**
Erfinder : **Mayer, Udo, Dr.
Max-Slevogt-Strasse 27
W-6710 Frankental (DE)**
Erfinder : **Oberlinner, Andreas, Dr.
Bruesseler Ring 53
W-6700 Ludwigshafen (DE)**

EP 0 304 613 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Benzopyranderivate der Formel I

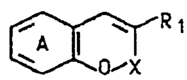

(I) ,

in der

$R^1$ für $C_1$-$C_8$-Alkyl, gegebenenfalls durch $C_1$-$C_4$-Alkoxy oder Halogen substituiertes Phenyl, 2- oder 4-Methylphenyl, 4-Ethylphenyl, 4-Isopropylphenyl, $C_1$-$C_5$-Alkoxy oder Halogen und

X für den Rest

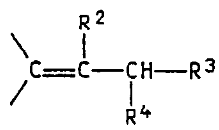

stehen, wobei

$R^2$ Wasserstoff oder zusammen mit $R^1$ gegebenenfalls durch $C_1$-$C_4$-Alkyl substituiertes $C_2$-$C_3$-Alkylen,

$R^3$ $C_1$-$C_4$-Alkoxyphenyl, dessen Alkylrest durch Phenyl substituiert ist, $C_1$-$C_4$-Mono oder Bis(cyanoalkyl)aminophenyl, Naphthyl, das durch $C_1$-$C_4$-Mono- oder Dialkylamino, Phenylamino oder N-($C_1$-$C_4$-Alkyl)-N-phenylamino substituiert ist, 1- oder 2-Phenylindol-3-yl, den Rest

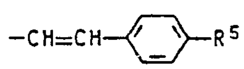

,

den Rest 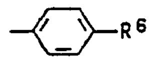 oder

den Rest 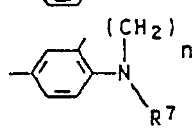 und

$R^4$ Hydroxy, $C_1$-$C_5$-Alkoxy, gegebenenfalls durch $C_1$-$C_4$-Alkyl, $C_1$-$C_4$-Alkoxy oder Halogen substituiertes Phenoxy, gegebenenfalls durch $C_1$-$C_4$-Alkyl oder Halogen substituiertes Phenylsulfonyl, Pyrrolidino, Piperidino, Morpholino oder den Rest einer C-H-aciden Verbindung aus der Gruppe, bestehend aus 2-(Pyrrolidino-, Piperidino- oder Morpholino-)cyclopent-1-en-1-yl oder -cyclohex-1-en-1-yl, gegebenenfalls in Ringposition 5 durch $C_1$-$C_4$-Alkyl mono- oder disubstituiertes Cyclohexan-1,3-dion-2-yl, Benzoylmethyl, Cyanomethyl, Nitromethyl, 2,4,6-Trihydroxypyrimid-5-yl, 1-Phenyl-3-methylpyrazol-5-on-4-yl, 5-Hydroxy-3,4-dichlorfuran-2-yloxy und dem Rest

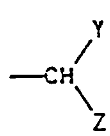

in dem Y und Z gleich oder verschieden sind und jeweils unabhängig voneinander fur Acetyl, Benzoyl, $C_1$-$C_5$-Alkoxycarbonyl oder Cyano stehen und in dem für den Fall, daß Y die Bedeutung von Cyano besitzt, Z auch für Methyl stehen kann, bedeuten und in der der Ring A gegebenenfalls durch einen Benzoring anelliert oder gegebenenfalls durch $C_1$-$C_4$-Alkyl, Chlor oder Brom substituiert oder in Ringposition 7 gegebenenfalls durch Hydroxy, $C_1$-$C_4$-Alkoxy, $C_1$-$C_5$-Mono- oder Dialkylamino, das jeweils wiederum durch Chlor oder Phenyl substituiert sein kann, Pyrrolidino, Piperidino oder Morpholino substituiert ist, wobei

$R^5$ für $C_1$-$C_8$-Alkyl oder $C_1$-$C_8$-Alkoxy, das gegebenenfalls jeweils durch Phenyl substituiert ist, $C_5$-$C_7$-Cycloalkyl, $C_5$-$C_7$-Cycloalkoxy, $C_1$-$C_4$-Mono- oder Dialkylamino, Phenylamino oder N-($C_1$-$C_4$-Alkyl)-N-phenylamino,

$R^6$ für den Rest

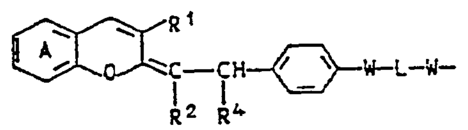,

wobei $R^1$, $R^2$, $R^4$ und der Ring A jeweils die obengenannte Bedeutung besitzen, W Sauerstoff oder $C_1$-$C_4$-Alkylimino und L $C_2$-C6-Alkylen oder

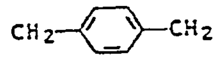 bedeuten,

$R^7$ für $C_1$-$C_4$-Alkyl und n für 2 oder 3 stehen, mit der Maßgabe, daß wenn der Ring A unsubstituiert und nicht benzoanelliert ist und $R^1$ Methyl und X den Rest

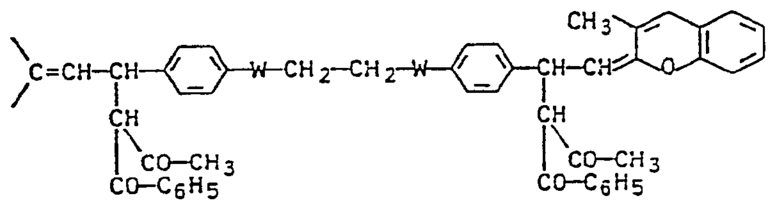

bedeutet, W nicht für Ethylimino steht.

Benzopyranderivate mit ähnlicher Struktur sind auch in der EP-A-236 973 beschrieben. Dabei handelt es sich um ein Dokument gemäß Art. 54 (3) EPÜ

Alle in den obengenannten Resten auftretenden Alkyl- und Alkylengruppen können sowohl geradkettig als auch verzweigt sein.

$R^1$ in Formel I bedeutet z.B. Methyl, Ethyl, Propyl, Isopropyl, Butyl, Isobutyl, tert -Butyl, Pentyl, Isopentyl, sec-Pentyl, tert-Pentyl, Hexyl, Heptyl, Octyl, 2-Ethylhexyl ; Phenyl, 4-Methoxyphenyl, 2-Ethoxyphenyl, 4-Chlorphenyl, 2,4-Dichlorphenyl ; Methoxy, Ethoxy, Propoxy, Isopropoxy, Butoxy ; Fluor, Chlor oder Brom,

$R^2$ in Formel I bedeutet z.B. Wasserstoff oder zusammen mit $R^1$ 1,2-Ethylen 1,2-Propylen, 1,3-Propylen oder 2-Methyl-1,3-propylen.

$R^3$ in Formel I bedeutet z.B. 4-Benzyloxyphenyl, 4-(1-Phenylethoxy)phenyl, 4-(2-Phenylethoxy)phenyl, 4-(Bis(2-cyanoethyl)amino)phenyl, 4-Methylaminonaphth-1-yl, 4-Ethylaminonaphth-1-yl, 4-Dimethylaminonaphth1-yl, 4-Diethylaminonaphth-1-yl, 4-Diisopropylaminonaphth-1-yl, 4-Dibutylaminonaphth-1-yl, 4-Phenylaminonaphth-1-yl, 4-(N-Methyl-N-phenylamino)naphth-1-yl, 4-(N-Ethyl-N-phenylamino)naphth-1-yl, 4-Methylstyryl, 4-Ethylstyryl, 4-Isopropylstyryl, 4-Butylstyryl, 4-Isopentylstyryl,4-Hexylstyryl, 4-Heptylstyryl, 4-Octylstyryl, 4-(2-Ethylhexyl)styryl, 4-Benzylstyryl, 4-(2-Phenylethyl)styryl, 4-Methoxystyryl, 4-Ethoxystyryl, 4-Propoxystyryl, 4-Butoxystyryl, 4-Benzyloxystyryl, 4-(2-Phenylethoxy)styryl, 4-Cyclopentylstyryl, 4-Cyclohexylstyryl, 4-Cycloheptylstyryl, 4-Cyclopentyloxystyryl, 4-Cyclohexyloxystyryl, 4-Cycloheptyloxystyryl, 4-Methylaminostyryl, 4-Ethylaminostyryl, 4-Dimethylaminostyryl, 4-Phenylaminostyryl, 4-(N-Methyl-N-phenylamino)styryl oder 4-(N-Ethyl-N-phenylamino)styryl, 1-Methyl-2,3-dihydroindol-5-yl, 1-Ethyl-2,3-dihydroindol-5-yl, 1-Methyl-1,2,3,4-tetrahydrochinolin-6-yl oder 1-Ethyl-1,2,3,4-tetrahydrochinolin-6-yl.

$R^4$ in Formel I bedeutet z.B Hydroxy ; Methoxy, Ethoxy, Propoxy, Isopropoxy, sec-Butoxy, Pentyloxy ; Phenoxy, 2-Methlyphenoxy, 4-Methylphenoxy, 3-Methoxyphenoxy, 4-Chlorphenoxy ; Phenylsulfonyl, 4-Methlyphenylsulfonyl, 4-Chlorphenylsulfonyl, Bis(acetyl)methyl, Bis(benzoyl)methyl, Bis(methoxycarbonyl)methyl, Bis(ethoxycarbonyl)methyl, Bis(cyano)methyl, Acetyl-benzoyl-methyl, Acetyl-methoxycarbonyl-methyl, Benzoyl-ethoxycarbonyl-methyl, Cyano-methoxycarbonyl-methyl, Cyano-ethoxycarbonyl-methyl oder 1-Cyanoeth-1-yl.

Der Ring A in Formel I kann beispielsweise in Ringposition 6 oder 8 durch Chlor oder Brom oder in Ringposition 6 und 8 durch Chlor substituiert sein Er kann weiterhin in Ringposition 7 beispielsweise Methyl Ethyl, Propyl, Isopropyl, Butyl ; Hydroxy ; Methoxy, Ethoxy, Isopropoxy ; Methylamino Ethylamino, Propylamino, Isopropylamino Butylamino, Benzylamino 2-Chlorethylamino. Dimethylamino, Diethylamino, Dibutylamino, Dibenzylamino, Methyl-ethyl-amino ; Pyrrolidino, Piperidino oder Morpholino als Substituenten aufweisen.

Außerdem kann er z.B. wie folgt benzoanelliert sein :

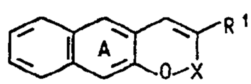 , 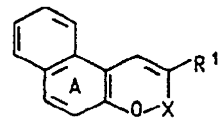

oder

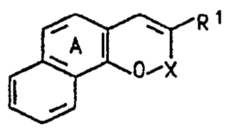

Bevorzugt sind solche Benzopyranderivate der Formel I, in der $R^1$ $C_1$-$C_5$-Alkyl, $R^2$ Wasserstoff, $R^3$ $C_1$-$C_4$-Alkoxyphenyl, dessen Alkylrest durch Phenyl substituiert ist und $R^4$ den Rest

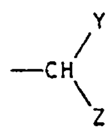 ,

in dem Y und Z jeweils die obengenannte Bedeutung besitzen.

Weiterhin sind Benzopyranderivate der Formel I bevorzugt, in der $R^3$ den Rest

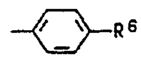 ,

wobei $R^6$ die obengenannte Bedeutung besitzt, und W Sauerstoff bedeuten.

Die erfindungsgemäßen Benzopyranderivate werden vorteilhaft erhalten, wenn man z.B. eine Benzopyryliumverbindung der Formel II

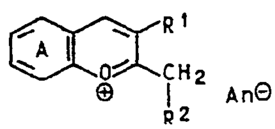 (II),

in der $R^1$, $R^2$ und der Ring A jeweils die obengenannte Bedeutung besitzen und An⊖ ein Anion bedeutet, mit einem Aldehyd der Formel III

$R^3$—CHO (III),

in der $R^3$ die obengenannte Bedeutung besitzt, im Molverhältnis 1 : 0,8 bis 1 : 1,2 in Gegenwart eines inerten Lösungsmittels bei einer Temperatur von 20 bis 120°C, vorzugsweise von 40 bis 80°C, umsetzt und in einer Folgestufe das resultierende Farbsalz der Formel IV

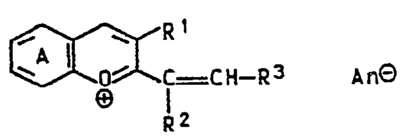 (IV),

in der $R^1$, $R^2$, $R^3$, An⊖ und der Ring A jeweils die obengenannte Bedeutung besitzen, mit einer Verbindung der Formel V

$R^4—H$ (V),

in der $R^4$ die obengenannte Bedeutung besitzt, im Molverhältnis 1 : 1,1 bis 1 : 2 in Gegenwart eines inerten Lösungsmittels und einer Base bei einer Temperatur von 20 bis 120°C, vorzugsweise 40 bis 80°C, zur Reaktion bringt.

Bei der Herstellung derjenigen Verbindungen der Formel I, in der $R^3$ für den Rest

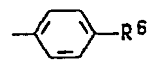

in dem $R^6$ die obengenannte Bedeutung besitzt, steht, d.h. die zwei Benzopyransysteme im Molekül aufweisen, setzt man die Benzopyryliumverbindung II zunächst mit einem entsprechenden Dialdehyd der Formel VI

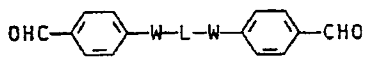 (VI),

in der L und W jeweils die obengenannte Bedeutung besitzen, und anschließend mit einer Verbindung der Formel V um. Hierbei muß bei den jeweiligen Molverhältnissen dem Umstand Rechnung getragen werden, daß es sich um bifunktionelle Verbindungen handelt.

Geeignete Anionen An⊖ sind z.B. Trichlorozinkat, Tetrachloroferrat(III), Hydrogensulfat Nitrat oder Halogenid, wie Chlorid oder Bromid. Besonders bevorzugt sind Trichlorozinkat oder Tetrachloroferrat(III).

Als inerte Lösungsmittel verwendet man zweckmäßig Alkohole, z.B. Methanol, Ethanol, Propanol, Isopropanol, Butanol oder Isobutanol. Gegebenenfalls werden auch Mischungen dieser Alkohole mit aromatischen Kohlenwasserstoffen, wie Toluol oder Xylol als Reaktionsmedium eingesetzt.

Geeignete Basen für die Umsetzung des Farbsalzes IV mit der Verbindung V sind beispielsweise Alkali- oder Erdalkalicarbonate, wie Natriumcarbonat, Kaliumcarbonat, Magnesiumcarbonat oder Calciumcarbonat ; Erdalkalioxide, wie Magnesiumoxid oder Calciumoxid ; oder Alkalialkanolate, wie Natrium- oder Kaliummethanolat, -ethanolat oder -butanolat. Im allgemeinen gibt man 1 bis 3 Mol Base, bezogen auf ein Mol Farbsalz, zu.

Die für das erfindungsgemäße Verfahren benötigten Benzopyryliumsalze II, Aldehyde III und Verbindungen IV sind bekannt.

Die erfindungsgemäßen Benzopyranderivate sind schwach farbige bis farblose Verbindungen, deren Lösungen in inerten organischen Lösungsmitteln im Kontakt mit Elektronenacceptoren, je nach der Substitution des Benzopyrans, Färbungen in gelben, orangen, roten, blauen oder grünen Farbtönen geben, Beispiele für Elektronenacceptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien, wie Kondensationsprodukte auf der Basis Phenolen und/oder Phenolsulfonsäuren, ferner Metalloxide oder -salze, wie Zinkoxid, Aluminiumoxid, Zinkchlorid, Eisenstearat oder Cobaltnaphthenat.

Aufgrund dieser Eigenschaften sind die neuen Verbindungen der Formel I als Farbbildner zur Verwendung in druck- und wärmeempfindlichen Aufzeichnungsmaterialien geeignet.

Für die Anwendung in druckempfindlichen Systemen werden die neuen Benzopyrane vorteilhaft in Form von Lösungen in organischen Lösungsmitteln, z.B. Chlorparaffine, partiell hydriertes Di- oder Terphenyl, Alkylbenzole, Alkylnaphthaline, alkylierte Dibenzylbenzole, Paraffinöl, Mineralöl oder auch übliche tiefer siedende Lösungsmittel, wie Xylol oder Toluol, in Mikrokapseln eingeschlossen und mit diesen der Träger, z.B. Papier beschichtet. Bei Druck tritt dann im Kontakt mit Elektronenacceptoren an der Druckstelle Farbbildung ein.

Geeignete Verfahren zur Herstellung von Mikrokapseln sind z.B. aus der US-A-2 8000 457, US-A-2 800 458, DE-A-2 119 933 oder EP-A-26 914 bekannt. Man kann die erfindungsgemäßen Verbindungen auch nach dem in der US-A-3 103 404 beschriebenen Verfahren in Wachs oder Öl-Wachsmischungen fein verteilen und mit diesen Mischungen Träger, wie Folien oder Papier beschichten. Man erhält druckempfindliche Materialien, die zum Durchschreiben auf mit Elektronenacceptorsubstanzen beschichteten Papieren geeignet sind und die nach Gebrauch wie Kohlepapier entfernt werden.

Die erfindungsgemäßen Benzopyrane können auch als Farbbildner in wärmeempfindlichen Aufzeichnungsmaterialien verwendet werden, die auf einem Träger ein Bindemittel, einen Farbbildner und eine Elektronenacceptorsubstanz enthalten. Der Aufbau solcher wärmeempfindlicher Aufzeichnungsmaterialien und die Zusammensetzung der durch Wärmeeinfluß die Farbe erzeugenden Schichten sind bekannt (z.B. DE-A-2 228

581 oder DE-A-2 110 854), ebenso die Verfahren und Vorrichtungen, mit denen die Farbbildung erreicht wird.

Die folgenden Beispiele sollen die Erfindung näher erläutern.

Beispiel 1

a. Synthese des Farbsalzes

33 g (0,1 Moll 2,3-Dimethylbenzopyryliumchlorozinkat und 21 g (0,1 Mol) 4-Benzyloxybenzaldehyd wurden in 100 ml Methanol 4 Stunden unter Rückfluß erhitzt. Der ausgefallene Farbstoff wurde nach dem Abkühlen abfiltriert und mit Methanol gewaschen.

Ausbeute : 40 g (88% der Theorie)
Schmp : 235-240°C Man erhielt ein Farbsalz der Formel

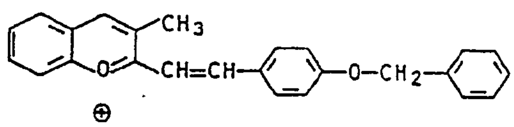

$ZnCl_3^{\ominus}$

(bzw. $ZnCl_4^{2\ominus}/2$)

b. Synthese des Farbbildners

13,7 g (0,03 Mol) des unter a. synthetisierten Farbsalzes und 4 g (0,04 Mol) Acetylaceton wurden in Gegenwart von 7 g Natriumcarbonat in 150 ml Methanol bei 45°C gerührt. Die Reaktionsmischung wurde in ein Gemisch aus 500 ml Toluol und 500 ml Wasser gegeben und verrührt. Die organische Phase wurde mit Aktivkohle behandelt, filtriert und unter vermindertem Druck eingedampft. Nach Zugabe von 100 ml Methanol kristallisierte der Rückstand durch und konnte abgesaugt werden.

Ausbeute : 10 g (74% der Theorie)

Man erhielt einen Farbbildner der Formel

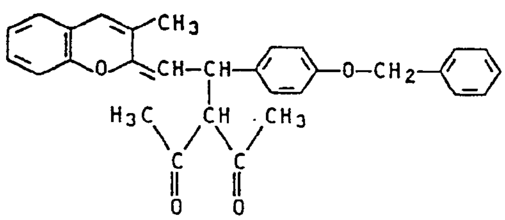

Die in der folgenden Tabelle 1 aufgeführten Verbindungen der Formel

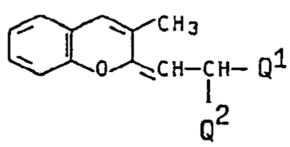

sowie die in Tabelle 2 aufgeführten Verbindungen werden in analoger Weise erhalten.

Tabelle 1

| Bsp. Nr. | $Q^1$ | $Q^2$ | Fp. [°C] | Farbe bzw. $\lambda$max [nm] |
|---|---|---|---|---|
| 2 | 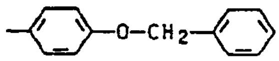 | 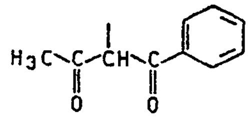 | 145 - 155 | 528 |
| 3 | 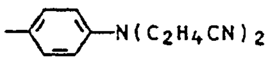 | 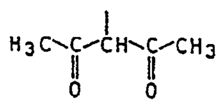 | 124 - 126 | 620 |
| 4 | 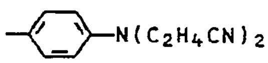 | 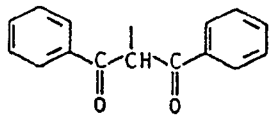 | 235 - 240 | 620 |
| 5 | 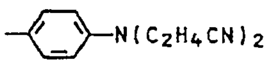 | 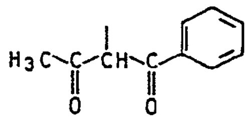 | 155 - 160 | 620 |
| 6 | 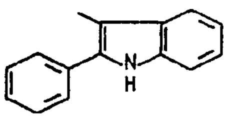 | 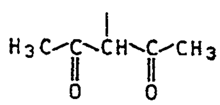 | 136 - 138 | 596 |
| 7 | 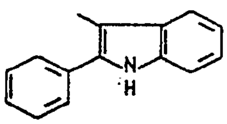 | 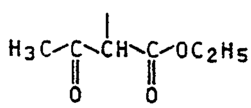 | 150 - 152 | 596 |
| 8 | 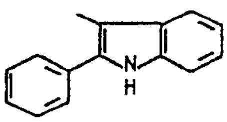 | 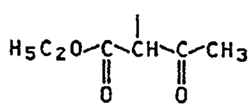 | 177 - 178 | 596 |
| 9 | 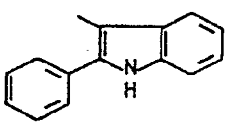 | 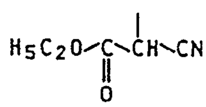 | 179 - 180 | 596 |
| 10 | 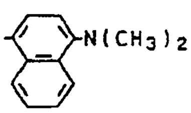 | 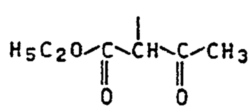 | 82 - 86 | blau |
| 11 | 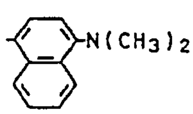 | 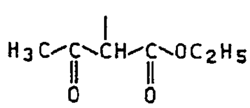 | 140 - 145 | blau |

Tabelle 2

| Bsp. Nr. | Formel | Fp. [°C] | Farbe bzw. $\lambda$max [nm] |
|---|---|---|---|
| 12 | 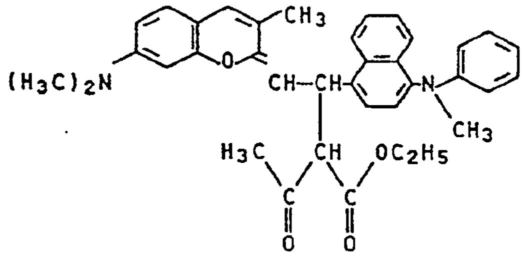 | 139 - 141 | grau |
| 13 | 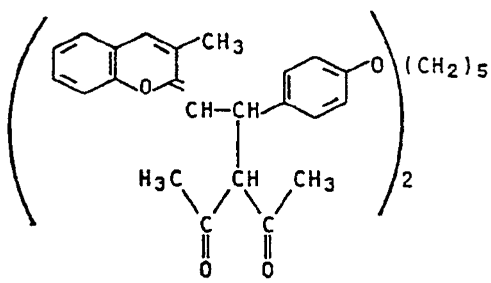 | Öl | rot |

## Ansprüche

1. Benzopyranderivate der Formel I

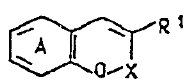 (I),

$R^1$ für $C_1$-$C_8$-Alkyl, gegebenenfalls durch $C_1$-$C_4$-Alkoxy oder Halogen substituiertes Phenyl, 2- oder 4-Methylphenyl, 4-Ethylphenyl, 4-Isopropylphenyl, $C_1$-$C_5$-Alkoxy oder Halogen und
X für den Rest

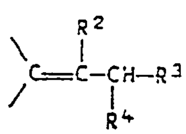 stehen, wobei

$R^2$ Wasserstoff oder zusammen mit $R^1$ gegebenenfalls durch $C_1$-$C_4$-Alkyl substituiertes $C_2$-$C_3$-Alkylen,
$R^3$ $C_1$-$C_4$-Alkoxyphenyl, dessen Alkylrest durch Phenyl substituiert ist, $C_1$-$C_4$-Mono- oder Bis(cyanoalkyl)aminophenyl, Naphthyl, das durch $C_1$-$C_4$-Mono- oder Dialkylamino, Phenylamino oder N-($C_1$-$C_4$-Alkyl)-N-phenylamino substituiert ist, 1- oder 2-Phenylindol-3-yl, den Rest

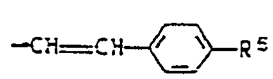, den Rest

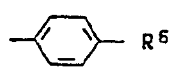 oder den Rest

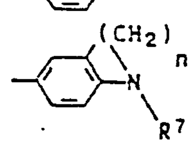 und

$R^4$ Hydroxy, $C_1$-$C_5$-Alkoxy, gegebenenfalls durch $C_1$-$C_4$-Alkyl, $C_1$-$C_4$-Alkoxy oder Halogen substituiertes Phenoxy, gegebenenfalls durch $C_1$-$C_4$-Alkyl oder Halogen substituiertes Phenylsulfonyl, Pyrrolidino, Piperidino, Morpholino oder den Rest einer C-H-aciden Verbindung aus der Gruppe, bestehend aus 2-(Pyrrolidino-, Piperidino- oder Morpholino-)cyclopent-1-en-1-yl oder -cyclohex-1-en-1-yl, gegebenenfalls in Ringposition 5 durch $C_1$-$C_4$-Alkyl mono- oder disubstituiertes Cyclohexan-1,3-dion-2-yl, Benzoylmethyl, Cyanomethyl, Nitromethyl, 2,4,6-Trihydroxypyrimid-S-yl, 1-Phenyl-3-methylpyrazol-5-on-4-yl, 5-Hydroxy-3,4-dichlorfuran-2-yloxy und dem Rest

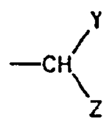

in dem Y und Z gleich oder verschieden sind und jeweils unabhängig voneinander für Acetyl, Benzoyl, $C_1$-$C_5$-Alkoxycarbonyl oder Cyano stehen und in dem für den Fall, daß Y die Bedeutung von Cyano besitzt, Z auch für Methyl stehen kann, bedeuten und in der der Ring A gegebenenfalls durch einen Benzoring anelliert oder gegebenenfalls durch $C_1$-$C_4$-Alkyl, Chlor oder Brom substituiert oder in Ringposition 7 gegebenenfalls durch Hydroxy, $C_1$-$C_4$-Alkoxy, $C_1$-$C_5$-Mono- oder Dialkylamino, das jeweils wiederum durch Chlor oder Phenyl substituiert sein kann, Pyrrolidino, Piperidino oder Morpholino substituiert ist, wobei

$R^5$ für $C_1$-$C_8$-Alkyl oder $C_1$-$C_8$-Alkoxy, das gegebenenfalls jeweils durch Phenyl substituiert ist, $C_5$-$C_7$-Cycloalkyl, $C_5$-$C_7$-Cycloalkoxy, $C_1$-$C_4$-Mono- oder Dialkylamino, Phenylamino oder N-($C_1$-$C_4$-Alkyl)-N-phenylamino,

$R^6$ für den Rest

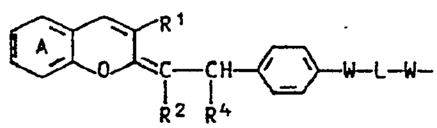

wobei $R^1$, $R^2$, $R^4$ und der Ring A jeweils die obengenannte Bedeutung besitzen, W Sauerstoff oder $C_1$-$C_4$-Alkylimino und L $C_2$-$C_6$-Alkylen oder

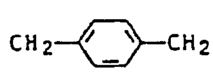 bedeuten.

bedeuten,

$R^7$ für $C_1$-$C_4$-Alkyl und

n für 2 oder 3 stehen, mit der Maßgabe, daß wenn der Ring A unsubstituiert und nicht benzoanelliert ist und $R^1$ Methyl und X den Rest

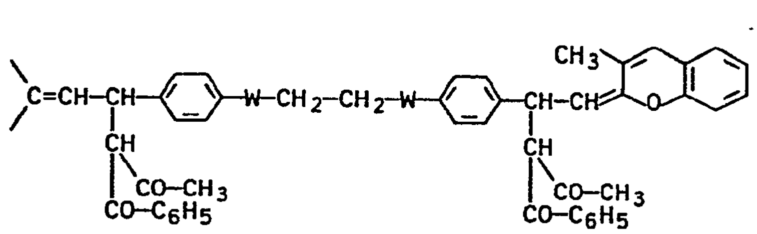

bedeutet, W nicht für Ethylimino steht.

2. Verwendung der Benzopyranderivate der Formel I gemäß Anspruch 1 als Farbbildner in druck- oder wärmeempfindlichen Aufzeichnungssystemen.

## Claims

1. A benzopyran derivate of the formula I

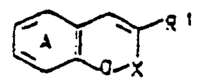

(I)

where

$R^1$ is $C_1$-$C_8$-alkyl, unsubstituted or $C_1$-$C_4$-alkoxy or halogen-substituted phenyl, 2- or 4-methylphenyl, 4-ethylphenyl, 4-isopropylphenyl, $C_1$-$C_5$-alkoxy or halogen and

X is the radical

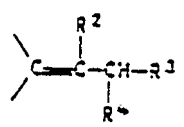

where

$R^2$ is hydrogen or together with $R^1$ is unsubstituted or $C_1$-$C_4$-alkyl-substituted $C_2$-$C_3$-alkylene,

$R^3$ is $C_1$-$C_4$-alkoxyphenyl whose alkyl radical is substituted by phenyl, $C_1$-$C_4$-mono- or -bis-(cyanoalkyl)-aminophenyl, naphthyl which is substituted by $C_1$-$C_4$-mono- or -di-alkylamino, phenylamino or N-($C_1$-$C_4$-alkyl)-N-phenylamino, 1- or 2-phenylindol-3-yl, the radical

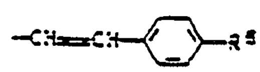

the radical 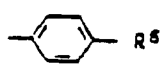

or the radical 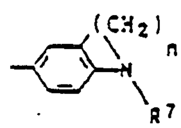 and

$R^4$ is hydroxyl, $C_1$-$C_5$-alkoxy, unsubstituted or $C_1$-$C_4$-alkyl-, $C_1$-$C_4$-alkoxy- or halogen-substituted phenoxy, unsubstituted or $C_1$-$C_4$-alkyl- or halogen-substituted phenylsulfonyl, pyrrolidino, piperidino, morpholino or the radical of an acidic C-H compound selected from the group consisting of 2-(pyrrolidino, piperidino or morpholino)cyclopent-1-en-1-yl or -cyclohex1-en-1-yl, cyclohexane-1,3-dion-2-yl which may be monosubstituted or disubstituted by $C_1$-$C_4$-alkyl in ring position 5, benzoylmethyl, cyanomethyl, nitromethyl, 2,4,6-trihydroxypyrimid-5-yl, 1-phenyl-3-methylpyrazol-5-on-4-yl, 5-hydroxy-3,4-dichlorofuran-2-yloxy and the radical

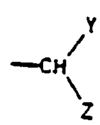

where Y and Z are identical or different and each is independently of the other acetyl, benzoyl, $C_1$-$C_5$-alkoxycarbonyl or cyano and where, if Y is cyano, Z may also be methyl, and where the ring A may be fused with a benzo ring or substituted by $C_1$-$C_4$-alkyl, chlorine or bromine or, in ring position 7, by hydroxyl, $C_1$-$C_4$-alkoxy, $C_1$-$C_5$-mono- or di-alkylamino (which in turn may be substituted by chlorine or phenyl), pyrrolidino, piperidino or morpholino,

$R^5$ being $C_1$-$C_8$-alkyl, $C_1$-$C_8$-alkoxy, which each may be substituted by phenyl, or being $C_5$-$C_7$-cycloalkyl, $C_5$-$C_7$-cycloalkoxy, $C_1$-$C_4$-mono- or di-alkylamino, phenylamino or N- ($C_1$-$C_4$-alkyl) -N-phenylamino,

$R^6$ being the radical

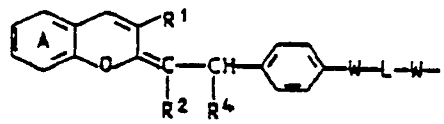

where $R^1$, $R^2$, $R^4$ and the ring A each have the above-mentioned meanings, W is oxygen or $C_1$-$C_4$-alkylimino, and L is $C_2$-$C_6$-alkylene or

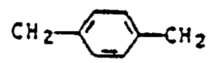

$R^7$ being $C_1$-$C_4$-alkyl and n being 2 or 3, with the proviso that when the ring A is unsubstituted and not benzofused and $R^1$ is methyl and X is the radical

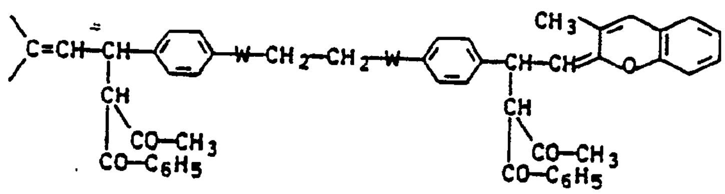

W is not ethylimino.

2. Use of a benzopyran derivative of the formula I as claimed in claim 1 as a dye-forming component in a pressure- or heat-sensitive recording system.

## Revendications

1. Dérivés de benzopyranne de formule I

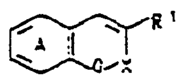

(I).

dans laquelle

$R^1$ est mis pour un groupement alkyle en $C_1$-$C_8$, phényle éventuellement substitué par un reste alcoxy en $C_1$-$C_4$ ou par un atome d'halogène, 2- ou 4-méthylphényle, 4-éthylphényle, 4- isopropylphényle, alcoxy en $C_1$-$C_5$ ou pour un atome d'halogène, et

X est mis pour le reste

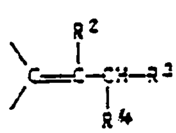

$R^2$ représentant un atome d'hydrogène ou, avec $R^1$, un reste alkylène en $C_2$-$C_3$ éventuellement substitué par un groupement alkyle en $C_1$-$C_4$,

$R^3$ représentant un groupement $C_1$-$C_4$-alcoxyphényle dont le reste alkyle est substitué par un radical phényle, un groupement $C_1$-$C_4$-mono- ou bis(cyanoalkyl)aminophényle, naphtyle qui est substitué par un reste $C_1$-$C_4$-mono- ou dialkylamino, phénylamino ou N-($C_1$-$C_4$-alkyl)-N-phénylamino, un groupement 1-ou 2-phenylindol-3-yle, le reste

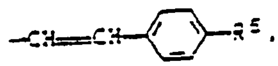 , le reste

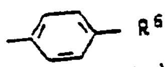 ou le reste

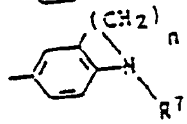 et

$R^4$ representent, un groupement hydroxy, alcoxy en $C_1$-$C_5$, phenoxy éventuellement substitué par un reste alkyle en $C_1$-$C_4$, alcoxy en $C_1$-$C_4$ ou par un atome d'halogène, un groupement phénylsulfonyle éventuellement substitué par un reste allyle en $C_1$-$C_4$ ou par un atome d'halogène, un groupement pyrrolidino, pipéridlno, morpholino ou le reste d'un composé acide C-H du groupe constitué par les groupements 2-(pyrrolidino-, pipéridino- ou morpholino-)cyclopent,-1-ène-1-yle ou -cyclohex-1-ène-1-yle, cyclohexane-1,3-dione-2-yle (éventuellement mono- ou disubstitué en position 5 du noyau par des groupements alkyle en $C_1$-$C_4$), benzoylméthyle, cyanométhyle, nitrométhyle, 2,4,6-trihydroxypyrimid-5-yle, 1-phényl-3-méthylpyrazol-5-one-4-yle, 5-hydroxy-3,4-dichlorofuranne-2-yloxy et par le reste

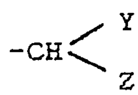

dans lequel Y et Z sont identiques ou différents et peuvent être mis chacun, indépendamment l'un de l'autre, pour un groupement acétyle, benzoyle, $C_1$-$C_5$-alcoxycarbonyle ou cyano et, dans le cas où Y a la signification d'un groupement cyano, Z peut être également mis pour un groupement méthyle, et dans laquelle
le noyau A est éventuellement condensé avec un noyau benzénique, est éventuellement substitué par un groupement alkyle en $C_1$-$C_4$ ou par un atome de chlore ou de brome ou est éventuellement substitué, en position 7 du noyau, par un groupement hydroxy, alcoxy en $C_1$-$C_4$, $C_1$-$C_5$-mono- ou dialkylamino (dont chacun peut être à son tour substitué par un atome de chlore ou un reste phényle), pyrrolidino, pipéridino ou morpholino,

$R^5$ mis pour un groupement alkyle en $C_1$-$C_8$ ou alcoxy en $C_1$-$C_8$ dont chacun est éventuellement substitué par un reste phényle, pour un groupement cycloalkyle en $C_5$-$C_7$, cycloalcoxy en $C_5$-$C_7$, $C_1$-$C_4$-mono- ou dialkylamino, phénylamino ou N-($C_1$-$C_4$-alkyl)-N-phénylamino,

$R^6$ étant mis pour le reste

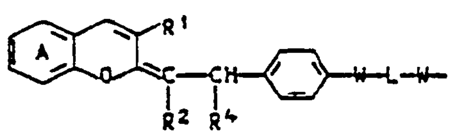 ,

$R^1$, $R^2$, $R^4$ et le noyau A ayant chacun la signification donnée précédemment, W représentant un atome d'oxygène ou un reste $C_1$-$C_4$-alkylimino et L représentant un groupement alkylène en $C_2$ à $C_8$ ou

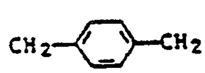

$R^7$ étant mis pour un groupement alkyle en $C_1$-$C_4$ et n étant mis pour 2 ou 3, étant spécifié que quand le noyau A est non substitué et non benzocondensé et que $R^1$ représente un groupement méthyle et X représente le reste

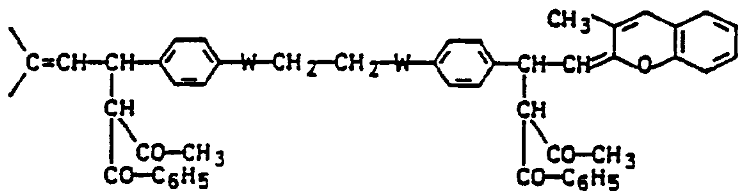

W n'est pas mis pour le groupement éthylimino.

2. Utilisation des dérivés de benzopyrranne de formule I selon la revendication 1 comme chromogène dans des systèmes d'enregistrement sensibles à la pression ou à la chaleur.